# EUROPÄISCHE PATENTANMELDUNG

(11) **EP 2 011 458 A1**
(43) Veröffentlichungstag der Anmeldung: **07.01.2009**
(21) Anmeldenummer: 07111960.6
(22) Anmeldetag: 06.07.2007
(51) Int. Cl.: A61F 9/007

(54) **Chirurgisches Instrument**

(71) Anmelder: WEFISinnovid GmbH, 50996 Köln (DE)
(72) Erfinder: Wernz, Dietrich, 53604 Bad Honnef (DE)
(74) Vertreter: Hilleringmann, Jochen

(57) **Zusammenfassung**

Das chirurgisches Instrument wie beispielsweise Phaco-Emulsifikationshandstück, ist versehen mit einem Gehäuse (22), einem Bearbeitungswerkzeug (12) zur Bearbeitung von menschlichem oder tierischem Gewebe, einer in dem Gehäuse (22) angeordneten Sonotrode (26), die ein Schwingungselement (46) und ein Spannelement (48) aufweist und einem Schwingungsantrieb (38) zum Versetzen des Schwingungselements (46) der Sonotrode (26) in Schwingungen, wobei der Schwingungsantrieb (38) durch das Spannelement (48) gegen das Schwingungselement (46) vorgespannt ist. Das Schwingungselement (46) der Sonotrode (26) ist als Spritzgussteil aus einem thermoplastischen Kunststoff ausgebildet.

## Beschreibung

Die Erfindung betrifft ein chirurgisches Instrument wie beispielsweise ein Phaco-Emulsifikationshandstück.

Es existieren die unterschiedlichsten Instrumente, die in der Chirurgie am menschlichen oder tierischen Körper eingesetzt werden. Eine Gruppe derartiger chirurgischer Instrumente weist Bearbeitungswerkzeuge auf, die in Schwingungen längs der Längsachse des Werkzeuges versetzt werden. Ein Beispiel für eine derartige Art von chirurgischem Instrument ist ein Phaco-Emulsifikationshandstück, wie es in der Augenchirurgie zur Zertrümmerung der Augenlinse verwendet wird. Dieses Instrument weist ein Gehäuse auf, an dem ein Bearbeitungswerkzeug angeordnet ist. In dem Gehäuse befindet sich eine Sonotrode, die über einen Schwingungsantrieb in Längsschwingungen, d.h. in Vor- und Zurückbewegungen längs ihrer Längsachse versetzt wird. Das Bearbeitungswerkzeug ist mit einem Ende der Sonotrode lösbar verbindbar.

Die Sonotrode weist ein Schwingungselement auf, das von dem Schwingungsantrieb in Schwingungen versetzt wird. Von dem Schwingungselement steht ein Spannelement ab, mit dem der Schwingungsantrieb gegen das Schwingelement mechanisch vorgespannt wird. Bei dem Schwingungsantrieb handelt es sich im Regelfall um einen Piezokeramikantrieb, Bei einem Phaco-Emulsifikationshandstück ist die Sonotrode, also das Schwingungs- und das Spannelement, von einem Lumen durchzogen, das der Absaugung von Infusionsflüssigkeit dient, die über eine Zuleitung bis zum Bearbeitungswerkzeug gelangt. Die Infusionsflüssigkeit wird dann zusammen mit Geweberesten über das Lumen abgesaugt.

Ein Emulsifikationshandstück der zuvor genannten Art ist beispielsweise in WO-A-03/073969 beschrieben.

Die Sonotrode bekannter Phaco-Emulsifikationshandstücke besteht im Regelfall aus Titan. Bei Titan handelt es sich jedoch um einen recht teuren Werkstoff, weshalb die Herstellungskosten bekannter Phaco-Emulsifikationshandstücke nicht unbeträchtlich sind.

Aufgabe der Erfindung ist es, ein chirurgisches Instrument, wie beispielsweise ein Phaco-Emulsifikationshandstück zu schaffen, das sich kostengünstig herstellen lässt.

Zur Lösung dieser Aufgabe wird mit der Erfindung ein chirurgisches Instrument, insbesondere Phaco-Emulsifikationshandstück vorgeschlagen, das versehen ist mit
- einem Gehäuse,
- einem Bearbeitungswerkzeug zur Bearbeitung von menschlichem oder tierischem Gewebe,
- einer in dem Gehäuse angeordneten Sonotrode, die ein Schwingungselement und ein Spannelement aufweist und
- einem Schwingungsantrieb zum Versetzen des Schwingungselements der Sonotrode in Schwingungen, wobei der Schwingungsantrieb durch das Spannelement gegen das Schwingungselement vorgespannt ist.

Erfindungsgemäß ist bei diesem chirurgischen Instrument vorgesehen, dass das Schwingungselement der Sonotrode als Spritzgussteil aus einem thermoplastischen Kunststoff ausgebildet ist.

Überraschenderweise hat sich durch Versuche ergeben, dass das Schwingungselement einer Sonotrode eines chirurgischen Instruments aus thermoplastischem Kunststoff gefertigt werden kann. Hier bieten sich insbesondere die technischen Thermoplaste und thermoplastischen Spezialkunststoffe an, wie sie in Römpp, Lexikon Chemie, Version 2.0, Stuttgart/New York, Georg Thieme Verlag 1999, beschrieben sind.

Aufgabe der Sonotrode und insbesondere des Schwingungselements der Sonotrode ist es, die von dem Schwingungsantrieb erzeugten Schwingungen als Körperschall bis zum Bearbeitungswerkzeug zu übertragen. Bisher war man davon ausgegangen, dass zum Zwecke dieser Körperschallübertragung ein metallischer Werkstoff, wie insbesondere Titan, Verwendung finden muss. Außerdem erwärmt sich das Schwingungselement und die gesamte Sonotrode während des Betriebs.

Überraschenderweise hat sich nun herausgestellt, dass hochtemperaturfeste Kunststoffe bzw. die technischen Thermoplaste ebenfalls als Materialien geeignet sind, die in Spritzgusstechnik zur Herstellung zumindest des Schwingungselements der Sonotrode verwendet werden können. Diese Klasse von Kunststoffen hält zum einen den erhöhten Temperaturen während des Betriebs der Sonotrode Stand und überträgt zum anderen den Körperschall, der durch den Schwingungsantrieb eingebracht wird.

Als thermoplastische Kunststoffe zur Herstellung zumindest des Schwingungselements einer Sonotrode kommen vorzugsweise Kunststoffe auf Polysulfon-Basis in Frage. Hier seien insbesondere PPS, PPSU, PES und PSU zu nennen. Weitere mögliche Kunststoffwerkstoffe, die sich zur Verwendung als Herstellungsmaterial für eine Sonotrode eignen, sind PEEK, PAI, PEI und APE.

Durch die Ausbildung des Schwingungselements der Sonotrode als Spritzgussteils aus thermoplastischem Kunststoff können die Herstellungskosten für die Sonotrode und damit für das gesamte chirurgische Instrument deutlich gesenkt werden. Die Sonotrode kann nunmehr insbesondere auch als Einweg-Bauteil ausgeführt werden, das nach seinem Gebrauch direkt entsorgt wird.

In vorteilhafter Weiterbildung der Erfindung ist ferner vorgesehen, dass das Spannelement aus einem metallischen Werkstoff besteht und durch Umspritzung des thermoplastischen Kunststoffs des Schwingungselements der Sonotrode mit diesem einstückig verbunden ist. Dabei entsteht insbesondere durch die Ausbildung von Hinterschnitten innerhalb des Bereichs des Spannelements, in dem dieses vom thermoplastischen Kunststoff des Schwingungselements umspritzt ist, eine formschlüssige Verbindung. Das Spannelement umfasst unter anderem eine Spannmutter, die auf das Außengewinde eines Schaftes aufgeschraubt ist, welcher von dem Schwingungselement absteht und in diesem kraftschlüssig verankert ist. Zwischen der Spannmutter und dem Schwingungselement befindet sich der Schwingungsantrieb aus beispielsweise piezokeramischen Elementen, die intermetierend strombeaufschlagt werden und sich dabei verformen, wodurch die Schwingung entsteht. Diese Art von Antrieben ist bei Phaco-Emulsifikationshandstücken grundsätzlich bekannt.

Als Material für das Spannelement, d.h. für den Schaft, kommt insbesondere Titan, Stahl oder Aluminium in Frage. Alternativ ist es möglich, auch dieses Spannelement, also den Schaft, aus einem thermoplastischen Kunststoff als Spritzgussteil herzustellen. In diesem Fall ist dann die gesamte Sonotrode als Spritzgussteil aus thermoplastischem Kunststoff ausgebildet.

Die Erfindung wurde vorstehend anhand der Verwendung der Sonotrode in einem chirurgischem Instrument beschrieben. Gegenstand der Erfindung selbst ist aber die Sonotrode mit einem Schwingungselement, das als Spritzgussteil aus einem thermoplastischen Kunststoff ausgebildet ist, wobei es für die Erfindung in erster Linie unerheblich ist, bei welchem chirurgischen Instrument oder bei welchem anderen technischen Instrument sie eingesetzt wird.

Die Erfindung wird nachfolgend anhand eines Ausführungsbeispiels und unter Bezugnahme auf die Zeichnung näher erläutert. Im Einzelnen zeigen dabei:
- Fig. 1: die Anordnung einer Phaco-Hohlnadel mit an dieser angeschlossenem Schlauch,
- Fig. 2: eine Querschnittsansicht eines Phaco-Emulsifikationshandstücks mit Phaco-Hohlnadel und Schlauch gemäß Fig. 1, und
- Fig. 3: eine Ansicht der zweiteiligen Sonotrode, die ein Schwingungselement aus einem hochtemperaturfesten Thermoplasten und ein Spannelement aus Metall aufweist.

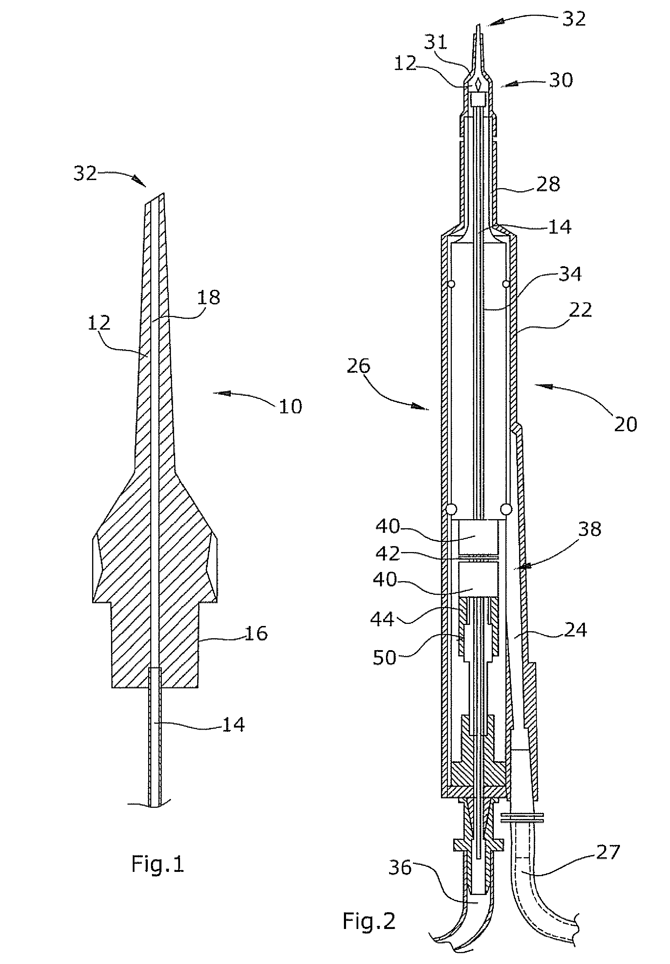

In Fig. 1 ist eine Anordnung 10 aus einer Phaco-Nadel 12 als Werkzeug für die Augenchirurgie mit einem daran angeschlossenen Aspirationsschlauch 14 gezeigt. Die Phaco-Nadel 12 ist an ihrem einen Ende mit einem Außengewinde 16 versehen und weist einen durchgehenden Kanal 18 auf. In diesem Kanal 18 ist am Gewindeende der Phaco-Nadel 12 der Aspirationsschlauch 14 fluiddicht und fest eingesetzt.

Gemäß Fig. 2 ist diese Anordnung 10 in ein Phaco-Emulsifikationshandstück 20 eingesetzt. Dieses Handstück 20 weist ein im Wesentlichen zylindrisches Gehäuse 22 mit einem in der Gehäusewand integrierten Infusionszuführkanal 24 und eine im Gehäuse 22 angeordnete längliche Sonotrode 26 auf. An den Infusionszuführkanal ist ein Schlauch 27 zum Zuführen von Infusionsflüssigkeit angeschlossen. Die Sonotrode 26 ist an ihrem vorderen Ende 29 mit der Phaco-Nadel 12 verschraubt. Zwischen dem Gehäuse 22 und der Sonotrode 26 erstreckt sich durch das Handstück 20 hindurch ein ringförmiger Kanal 28 für die Infusionsflüssigkeit, die am vorderen Ende 30 des Gehäuses 22 im Bereich der Phaco-Nadel 12 austritt. Auf das vordere Ende 30 des Gehäuses 22 ist ein Überzug 31 aufgeschoben, der bis zum vorderen Ende 32 der Phaco-Nadel 12 reicht. Der an die Phaco-Nadel 12 angeschlossene Aspirationsschlauch 14 erstreckt sich durch einen Lumen 34 durch die Sonotrode 26 und den übrigen Teil des Gehäuses 22, an den eine Aspirationsleitung 36 angeschlossen ist, die zu einem Absauggerät führt.

Wie in Fig. 2 des weiteren zu erkennen ist, findet sich in dem Gehäuse 22 ein Schwingungsantrieb 38, der zwei oder mehrere piezokeramische Elemente 40 mit dazwischenliegenden elektrischen Kontaktierungen 42 aufweist, durch die sich ein Teil der Sonotrode 26 erstreckt. Die piezokeramischen Elemente 40 werden mittels einer Spannschraube 44, die in Gewindeeingriff mit der Sonotrode 26 stehen, gegen diese verspannt. Wenn nun die piezokeramischen Elemente 40 von elektrischem Strom durchflossen werden, so erzeugt der Schwingungsantrieb 38 Schallwellen, die von der Sonotrode 26 ausgehend vom Schwingungsantrieb 38 bis zum vorderen Ende 29 übertragen und dort in das Werkzeug, d.h. die Phaco-Nadel 12, eingeleitet werden.

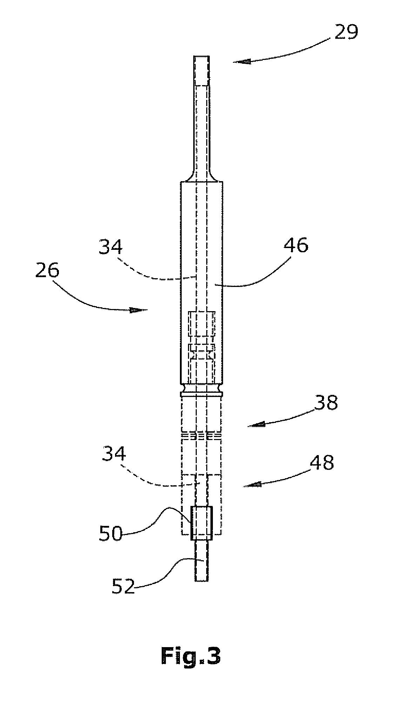

Die Sonotrode 26 ist als Einzelteil in Fig. 3 gezeigt. Sie umfasst ein Schwingungselement 46 und ein Spannelement 48. Während das Spannelement 48 aus einem metallischen Werkstoff besteht, ist das Schwingungselement 46 als Spritzgussteil aus einem hochtemperaturfesten Thermoplasten (Spezialkunststoff bzw. Hochleistungskunststoff gemäß Definition in Römpp, Lexikon Chemie) ausgebildet. Das Schwingungselement 46 und das Spannelement 48 sind von dem Lumen 34 durchzogen. Die Verbindung zwischen beiden Elementen der Sonotrode 26 erfolgt durch einen Formschluss durch teilweises Umspritzen des Spannelements 48 durch das thermoplastische Material, aus dem das Schwingungselement 46 besteht. Hierdurch kommt es zu einer auszugsfesten Verbindung zwischen dem Spannelement 48 und dem Schwingungselement 46. Der Formschluss nimmt die Zugkräfte auf, die bei aktiviertem Schwingungsantrieb 38 durch diesen und durch die über Spannschraube 44, die das Außengewinde 50 des Schaftes 52 des Spannelements 48 aufgebracht werden, zeugen.

Der besondere Vorteil des hier vorgestellten Phaco-Emulsifikationshandstücks 20 besteht in der kostengünstigen Herstellung der Sonotrode 26, die zumindest bezüglich ihres Schwingungselements 46 aus einem hochtemperaturfesten Thermoplasten, wie beispielsweise PPS, PPSU, PES oder PSU besteht. Nach Gebrauch des Phaco-Imulsifikationshandstücks 20 wird die Phaco-Nadel 12 abgeschraubt und zusammen mit dem Aspirationsschlauch 14 aus dem Handstück 20 entfernt. Nach Sterilisation des Handstücks kann dieses bestückt mit einer neuen Phaco-Nadel nebst Aspirationsschlauch wieder verwendet werden.

Durch die spezielle Konstruktion des Handstücks 20 mit einfach austauschbarer Phaco-Nadel und Aspirationsschlauch ist eine besonders einfache Möglichkeit geschaffen, mit minimalem Aufwand das Handstück 20 zu sterilisieren.

## Patentansprüche

1. Chirurgisches Instrument wie beispielsweise Phaco-Emulsifikationshandstück, mit
- einem Gehäuse (22),
- einem Bearbeitungswerkzeug (12) zur Bearbeitung von menschlichem oder tierischem Gewebe,
- einer in dem Gehäuse (22) angeordneten Sonotrode (26), die ein Schwingungselement (46) und ein Spannelement (48) aufweist und
- einem Schwingungsantrieb (38) zum Versetzen des Schwingungselements (46) der Sonotrode (26) in Schwingungen, wobei der Schwingungsantrieb (38) durch das Spannelement (48) gegen das Schwingungselement (46) vorgespannt ist,
**dadurch gekennzeichnet ,**
- **dass** das Schwingungselement (46) der Sonotrode (26) als Spritzgussteil aus einem thermoplastischen Kunststoff ausgebildet ist.

2. Chirurgisches Instrument nach Anspruch 1, **dadurch gekennzeichnet, dass** das Spannelement (48) einen von dem Schwingungselement (46) abstehenden Schaft (52) mit einem Außengewinde (50) in zumindest einem Teil seiner Außenfläche aufweist.

3. Chirurgisches Instrument nach Anspruch 1 oder 2, **dadurch gekennzeichnet, dass** das Spannelement (48) einen metallischen Werkstoff aufweist und durch teilweises Umspritzen kraftschlüssig mit dem Schwingungselement (46) verbunden ist.

4. Chirurgisches Instrument nach Anspruch 3, **dadurch gekennzeichnet, dass** der metallische Werkstoff Titan, Stahl oder Aluminium aufweist.

5. Chirurgisches Instrument nach einem der Ansprüche 1 bis 4, **dadurch gekennzeichnet, dass** die Sonotrode von einem Lumen (34) durchzogen ist, an dessen einem Ende (29) ein eine Hohlnadel aufweisendes Werkzeug (12) und an dessen anderem Ende eine Aspirationsleitung (36) anschließbar ist.

6. Chirurgisches Instrument nach einem der Ansprüche 1 bis 5, **dadurch gekennzeichnet, dass** der thermoplastische Kunststoff ein Kunststoff auf Polysulfon-Basis wie z.B. PPS, PES oder PSU ist.
